# EUROPEAN PATENT APPLICATION

(11) **EP 1 564 297 A1**
(43) Date of publication of application: **17.08.2005**
(21) Application number: 04075510.0
(22) Date of filing: 17.02.2004
(51) Int. Cl.: C12N 15/82, C12N 9/02, C12N 5/10, A01H 5/00, C12N 15/53

(54) **Control of programmed cell death (PCD) in plants**

(71) Applicant: Nederlandse Organisatie voor Toegepast-Natuurwetenschappelijk Onderzoek TNO, 2628 VK Delft (NL)
(72) Inventor: Korthout, Henrie A.A.J, 1544 PT Zaanijk (NL); Caspers, Martinus Petrus Maria, 2289 EG, Rijswijk (NL); Wang, Mei, 2341 BV Oegstgeest (NL)
(74) Representative: Prins, Adrianus Willem, Mr. Ir.

(57) **Abstract**

The invention relates to a method of controlling programmed cell death in plants. In one aspects the present invention relates to such a method wherein a transgenic plant is produced that is less susceptible to stress-induced programmed cell death. The invention thus provides a method for controlling programmed cell death in a plant or plant-part comprising modifying the activity and/or expression of 2-oxoglutarate dehydrogenase-homologous protein in the cells of said plant or plant-part.

## Description

### FIELD OF THE INVENTION

The invention relates to a method of controlling programmed cell death in plants and to a plant, wherein the occurrence of programmed cell death is thus controlled. In one aspect the present invention relates to such a method wherein a transgenic plant is produced that is less susceptible to biotic and abiotic stress-induced programmed cell death. The invention further relates to transgenic plants capable of overexpressing a gene involved in programmed cell death, to gene constructs capable of overexpressing a gene involved in programmed cell death and to vectors comprising such constructs.

### BACKGROUND OF THE INVENTION

Apoptosis or programmed cell death (PCD), a form of cell death described by Kerr and Wyllie some 20 years ago, has generated considerable interest in recent years. The mechanisms by which this mode of cell death takes place are known to occur in both animal and plant cells but have been examined in detail only in animal cells. Extracellular signals and intracellular events have been investigated because the pharmacological modulation of programmed cell death is of considerable clinical interest. Attempts to influence programmed cell death have been stimulated by the fact that it is reduced (like in cancer) or increased (like in neurodegenerative diseases) in several clinical situations. Pharmaceuticals that can modify programmed cell death are likely to be potentially useful drugs.

PCD is involved in the elimination of appropriate cells during developmental processes or in response to environmental cues. It is an active process, in which gene expression is intimately associated with the events leading to the orderly disassembly and death of the cells, and it is morphologically accompanied by condensation, shrinkage and fragmentation of the cytoplasm and nucleus. Biochemically, PCD is characterized by fragmentation of the nuclear DNA, and the induction of cysteine proteases and endonucleases.

Due to its relationship with cancer, most of the scientific investigation relating to programmed cell death has involved PCD in mammalian cells. The role of PCD in plant systems has not been studied extensively. In plants, PCD is believed to be involved in processes such as e.g., removal of the suspensor cells during the development of an embryo, the elimination of aleurone cells after germination of monocotyledonous seeds; the elimination of the root cap cells after seed germination and seedling growth; cell death during cell specialization as seen in development of xylem tracheary elements or trichomes, leaf senescence and floral organ aborting in unisexual flowers. Also the formation of aerochyma in roots under hypoxic conditions and the formation of leaf lobes or perforations in some plants seem to involve PCD. The restricted lesion occurring at the site of entry of an avirulent pathogen in plants, i.e. the hypersensitive response, is another example of PCD in response to an environmental cue.

Preliminary comparisons between plant and mammalian PCD mechanisms suggest some similarities in the mechanisms. The potential similarities include: an oxygen requirement; activation by hydrogen peroxide; a role for calcium in the activation process; a transcription requirement;-a dephosphorylation requirement and proteolytic and nucleolytic enzyme involvement. Despite the availability of the complete *Arabidopsis* genome sequence, plant orthologues to the key animal cell death proteins, including p53, the *bcl*-2 anti-apoptotic proteins and caspases (proteases), have not been identified. Further, there is a general consensus that reactive oxygen species (ROS) trigger PCD in plant cells. For instance, it was determined that during apoptosis in plants, NADPH oxidoreductase is up-regulated, which generates ROS such as triplet (³O₂) or singlet oxygen (¹O₂), superoxide anion (O₂^{.}-), hydroxyl radical (·OH), nitric oxide (NO·), peroxynitrite (ONOO-), hypochlorous acid (HOCl), hydrogen peroxide (H₂O₂), alkoxyl radical (LO·), and peroxyl radical (LO₂). Whether accumulation of ROS is specific only for those cells destined to undergo PCD, or alternatively, that cells destined to die do not invoke protective mechanisms against ROS is presently unknown.

Poly(ADP-ribose) polymerase (PARP) has been implicated as one of the enzymes in the apoptotic pathways induced by DNA damaging agents or oxidative stress in plants. In animals, pharmacological or genetic inhibition of PARP-1 during conditions of cellular stress, *e.g*. energy failure or oxidative stress, was found to be capable of preventing the occurrence of PCD, whereas in plants, such results seem to depend on the severity of the insult (Amor et al., FEBS Letters Vol. 440 (1-2) pp. 1-7). Therefore, controlling PCD in plants through the control of PARP activity has proven difficult. Controlling PCD by controlling PARP proteins is described by the patent application US2001011381 (De Block, Babiychuk and Kushnir).

The identification of key physiological mechanisms involved in PCD in plants may result in the development of methods with which the process may be controlled. Such methods may be used to either stimulate or repress PCD in plants. For instance control of PCD may be used to protect plants against stresses that normally induce PCD and that would result in damaged crops.

Thus, there is great need for tools capable of controlling PCD in plants in general, and in particular of preventing PCD in plants.

The present inventors have found that regulation of 2-oxoglutarate dehydrogenase activity can be used to control programmed cell death in plants. This finding now provides an additional control method.

### SUMMARY OF THE INVENTION

The present invention provides a method for controlling programmed cell death in a plant or plant-part comprising modifying the activity and/or expression of 2-oxoglutarate dehydrogenase-homologous protein in the cells of said plant or plant-part.

Preferably, in a method of the invention for controlling programmed cell death in a plant or plant-part, the activity of 2-oxoglutarate dehydrogenase-homologous protein and/or expression is up-regulated, most preferably resulting in the overexpression of 2-oxoglutarate dehydrogenase or results of higher activities of. 2-oxoglutarate dehydrogenase. In yet another preferred embodiment, said activity and/or expression is modified in the mitochondria of said cells..

One embodiment of a method of the invention for controlling programmed cell death in a plant or plant-part relates to a method that involves or comprises the occurrence of at least one mutation in the genotype of said plant or plant-part. Another embodiment relates to a method wherein the occurrence of at said least one mutation does not involve the insertion of foreign genetic material in the plant or rearrangement of genetic material within the plant. The advantage of such a method is that, because it does not involve transformation, the use of the method is not complicated by the issues of biosafety and bioethics.

Yet another embodiment of a method of the invention for controlling programmed cell death in a plant or plant-part relates to a method wherein the promoter of the gene encoding the 2-oxoglutarate dehydrogenase-homologous protein is replaced.

Still another embodiment relates to a method for controlling programmed cell death in a plant or plant-part wherein said method further involves marker-assisted selection and/or breeding. In a preferred embodiment thereof, the method involves allowing or inducing the occurrence of at least one mutation in the genotype of said plant or plant-part, and selecting plants modified in the activity and/or expression of 2-oxoglutarate dehydrogenase-homologous protein by using specific markers.

In another aspect, the present invention relates to a plant, wherein the occurrence of programmed cell death is controlled by a method according to the invention.

In another aspect, the present invention provides a method for preventing programmed cell death in a plant or plant-part comprising providing the cells of said plant or plant-part with a polynucleotide construct, comprising a recombinant polynucleotide for overexpression of a 2-oxoglutarate dehydrogenase-homologous protein encoding gene, and which polynucleotide comprises in operable linkage:
(a) a promoter that is functional in plants;
(b) said 2-oxoglutarate dehydrogenase-homologous protein encoding gene;
(c) a terminator; and, optionally,
(d) a screenable or selectable marker gene operably linked to regulatory sequences for expression. In a preferred such method, said 2-oxoglutarate dehydrogenase-homologous protein encoding gene is a heterologous gene. In other embodiment of such a method, said promoter is an inducible promoter. In yet other embodiments, promoters may be tissue specific promoters, such as fruit-, root-, leaf- or seed-specific promoters.

In another aspect, the present invention provides a transgenic plant or plant-part obtainable by a method according to the invention.

In yet another aspect, the present invention provides a polynucleotide construct, comprising a recombinant polynucleotide for overexpression of a 2-oxoglutarate dehydrogenase-homologous protein encoding gene, and which polynucleotide comprises in operable linkage:
(a) a promoter that is functional in plants;
(b) said 2-oxoglutarate dehydrogenase-homologous protein encoding gene;
(c) a terminator; and, optionally,
(d) a screenable or selectable marker gene operably linked to regulatory sequences for expression. In a preferred polynucleotide construct of the invention, the 2-oxoglutarate dehydrogenase-homologous protein encoding gene is a heterologous gene. The promoter may for instance be an inducible promoter. In other embodiments the promoter my for instance be a tissue specific promoter, such as a fruit-, root-, leaf- or seed-specific promoter.

In still further aspects, the present invention provides a vector comprising a polynucleotide construct according to the invention, an Agrobacterium strain or any other microbial strain comprising a vector according to the invention and a transgenic plant comprising a polynucleotide construct according to the invention.

In yet another aspect, the present invention provides a method for producing a transgenic plant comprising introducing into the genome of a plant a vector according to the invention, preferably said vector is introduced into an ancestor plant, and said transgenic plant is produced from said ancestor plant. Alternatively, said vector may be introduced into a plant-part thereby producing a transformed plant-part, followed by regenerating said transgenic plant from said transformed plant-part.

### DETAILED DESCRIPTION OF THE INVENTION

A "plant" refers to any plant, particularly to fruit and seed plants.

"Plant cell" refers to the structural and physiological unit of the plant, comprising a protoplast and a cell wall. The plant cell may be in form of an isolated single cell or a cultured cell, or as a part of higher organized unit such as, for example, a plant tissue, or a plant organ.

"Plant-part" refers to leaves, stems, roots, seeds, flowers or flower parts, fruits, pollen, pollen tubes, ovules, embryo sacs, egg cells, zygotes, embryos, seeds, cuttings, cell or tissue cultures, or any other part or product of a plant.

"Recombinant DNA technology" refers to procedures used to join together DNA sequences as described, for example, in Sambrook et al., 1989, Cold Spring Harbor, NY: Cold Spring Harbor Laboratory Press.

"Stress" refers to both biotic and a-biotic stress

"Transformation" refers to introduction of a nucleic acid into a cell such as for instance by the stable integration of a DNA molecule into the genome of an organism of interest.

"Expression" refers to the transcription and/or translation of an endogenous gene or a transgene in plants. In the case of polynucleotide constructs, for example, expression may refer to the transcription of the polynucleotide DNA alone as well as to the transcription and translation process collectively.

"Polynucleotide construct" as used herein means a recombinant nucleotide sequence capable of directing expression of a particular coding nucleotide sequence in an appropriate host cell, and comprising a promoter operably linked to the nucleotide sequence of interest which is optionally operably linked to 3' sequences, such as 3' regulatory sequences or termination signals. It also typically comprises sequences required for proper translation of the nucleotide sequence. The coding region usually codes for a protein of interest but may also code for a functional RNA of interest, for example antisense RNA or a nontranslated RNA that, in the sense or antisense or both directions, inhibits expression of a particular gene, e.g., antisense RNA or RNAi. The polynucleotide construct comprising the nucleotide sequence of interest may be chimeric, meaning that the nucleotide sequence is comprised of more than one nucleotide sequences of distinct origin which are fused together by recombinant DNA techniques resulting in a nucleotide sequence which does not occur naturally, and which particularly does not occur in the plant to be transformed. The polynucleotide construct may also be one which is naturally occurring but has been obtained in a recombinant form useful for heterologous expression. Typically, however, the polynucleotide construct is heterologous with respect to the host. The expression of the nucleotide sequence in the polynucleotide construct may be under the control of a constitutive or tissue-specific promoter or of an inducible promoter which initiates transcription only when the host cell is exposed to some particular external stimulus. In the case of a multicellular organism, such as a plant, the promoter can also be specific to a particular tissue or organ or stage of development. A nuclear polynucleotide construct is usually inserted into the nuclear genome of a plant and is capable of directing the expression of a particular nucleotide sequence from the nuclear genome of said plant.

"Regulatory elements" or "regulatory sequences" refer to sequences involved in conferring the expression of a coding nucleotide sequence. Regulator sequences comprise a promoter operably linked to the nucleotide sequence of interest and, optionally, 3' sequences, such as 3' regulatory sequences or termination signals. They also typically encompass sequences required for proper translation of the nucleotide sequence.

A regulatory nucleotide sequence is said to be "operably linked to" or "associated with" a nucleotide sequence that codes for an RNA or a protein if the two sequences are situated such that the regulatory nucleotide sequence affects expression of the coding nucleotide sequence.

A "promoter" refers to a DNA sequence that initiates transcription of an associated nucleotide sequence. The promoter region may also include elements that act as regulators of gene expression such as activators, enhancers, and/or repressors.

"Gene" refers to a coding sequence and associated regulatory sequences wherein the coding sequence is transcribed into RNA such as mRNA, rRNA, tRNA, snRNA, sense RNA or antisense RNA. Further elements that may be present in a gene are, for example, introns.

"Heterologous" as used herein means of different natural or of synthetic origin. For example, if a host cell is transformed with a nucleic acid sequence that does not occur in the untransformed host cell, that nucleic acid sequence is said to be heterologous with respect to the host cell. The transforming nucleic acid may comprise a heterologous promoter, heterologous coding sequence, or heterologous termination sequence. Alternatively, the transforming nucleic acid may be completely heterologous or may comprise any possible combination of heterologous and endogenous nucleic acid sequences. Similarly, heterologous refers to a nucleotide sequence derived from and inserted into the same natural, original cell type, but which is present in a non-natural state, e.g. a different copy number, or under the control of different regulatory elements.

"Overexpression" refers to a level of transcription and/or translation of an endogenous gene or a transgene in plants which exceeds the normal, natural, wild-type or untransformed level or demand thereof and may lead to physiological changes or altered phenotype of a plant. In the context of polynucleotide constructs, for example, overexpression may result in the overproduction of transcript and protein.

"Overproduction" refers to a level of production of a protein which exceeds the demand and may lead to physiological changes or altered phenotype of a plant.

A protein or RNA is said to be "produced at enhanced level" or "overproduced" if the concentration of the said protein in the cell where it is produced is at least 20% higher than that in the original cell, i.e. a cell of the original plant line which has now been transformed. Similarly, a plant, a plant tissue or a plant cell is defined as having an "enhanced level of resistance" to a PCD inducing condition if it can withstand a 20% enhanced effect of the same inducing condition, without detectable damage, than the original plant, plant tissue or plant cell. Overproduction of a molecule in a cell can be achieved via both traditional mutation and selection techniques and genetic modification methods using recombinant DNA technology.

A "screenable marker gene" refers to a gene whose expression does not confer a selective advantage to a transformed cell, but whose expression makes the transformed cell phenotypically distinct from untransformed cells, and which thus can be used to discriminate transformed from untransformed cells.

A "selectable marker gene" refers to a gene whose expression in a plant cell gives the cell a selective advantage. The selective advantage possessed by the cells transformed with the selectable marker gene may be due to their ability to grow in the presence of a negative selective agent, such as an antibiotic or an herbicide, compared to the growth of non-transformed cells. The selective advantage possessed by the transformed cells, compared to non-transformed cells, may also be due to their enhanced or novel capacity to utilize an added compound as a nutrient, growth factor or energy source. Selectable marker gene also refers to a gene or a combination of genes whose expression in a plant cell gives the cell both a negative and a positive selective advantage.

A "2-oxoglutarate dehydrogenase-homologous protein" is defined herein as an enzyme having 2-oxoglutarate dehydrogenase activity, i.e. which can convert 2-oxoglutarate (α-ketoglutarate) to succinyl-CoA, carbon dioxide and NADH, optionally in association with dihydrolipoamide succinyltransferase (E2) and/or lipoamide dehydrogenase, and optionally in the presence of TPP cofactor. The term "2-oxoglutarate dehydrogenase-homologous protein" as used herein also includes functional variants and derivatives of the said protein.

A "functional variant" or a "functional derivative" of a protein is a protein the amino acid sequence of which can be derived from the amino acid sequence of the original protein by the substitution, deletion and/or addition of one or more amino acid residues in a way that, in spite of the change in the amino acid sequence, the functional variant retains at least a part of at least one of the biological activities of the original protein that is detectable for a person skilled in the art. A functional variant is generally at least 50% homologous (preferably the amino acid sequence is at least 50% identical), advantageously at least 70% homologous and even more advantageously at least 90% homologous to the protein from which it can be derived. A functional variant may also be any functional part of a protein; the function in the present case being particularly but not exclusively 2-oxoglutarate conversion. Preferably the amino acid sequence differs from SEQ ID No 1 mainly or only by conservative substitutions. More preferably the protein comprises an amino acid sequence having 90% or more, still more preferably 95%, sequence identity with SEQ ID No 1 and optimally 100% identity with those sequences. "Functional" as used herein means functional in plants.

The term "nucleotide sequence homology" as used herein denotes the presence of homology between two (poly)nucleotides. Polynucleotides have "homologous" sequences if the sequence of nucleotides in the two sequences is the same when aligned for maximum correspondence. Sequence comparison between two or more polynucleotides is generally performed by comparing portions of the two sequences over a comparison window to identify and compare local regions of sequence similarity. The comparison window is generally from about 20 to 200 contiguous nucleotides. The "percentage of sequence homology" for polynucleotides, such as 50, 60, 70, 80, 90, 95, 98, 99 or 100 percent sequence homology may be determined by comparing two optimally aligned sequences over a comparison window, wherein the portion of the polynucleotide sequence in the comparison window may include additions or deletions (i.e. gaps) as compared to the reference sequence (which does not comprise additions or deletions) for optimal alignment of the two sequences. The percentage is calculated by: (a) determining the number of positions at which the identical nucleic acid base occurs in both sequences to yield the number of matched positions; (b) dividing the number of matched positions by the total number of positions in the window of comparison; and (c) multiplying the result by 100 to yield the percentage of sequence homology. Optimal alignment of sequences for comparison may be conducted by computerized implementations of known algorithms, or by inspection.

Algorithms and software suitable for use in aligning sequences for comparison and calculation of sequence homology or identity will be known to those skilled in the art. Significant examples of such tools are the Pearson and Lipman search based FASTA and BLAST programs, details of these may be found in Altschul *et al* (1997), Nucleic Acid Res. 25:3389-3402; Altschul *et al* (1990), J. Mol. Biol. 215: 403-10; Pearson and Lipman (1988), Proc. Natl. Acad. Sci. USA 85:2444-8; Lipman and Pearson (1985), Science 227:1435- 41). Other suitable programs include the PILEUP, LINEUP, GAP and BESTFIT programs in the GCG® Wisconsin Package® of the University of Wisconsin Genetics Computer Group, Madison, WI, USA, now offered through Accelrys Inc. Details of the above programs are available on the internet through 'http://www.ncbi.nlm.nih.gov/BLAST' or mirror sites and "http://www.accelrys.com/products/gcg_wisconsin_package". Thus such homology and identity percentages can be ascertained using publicly or commercially available software packages or by computer servers on the internet.

By the term "identity" is meant that the stated percentage of the claimed amino acid sequence or base sequence is to be found in the reference sequence in the same relative positions when the sequences are optimally aligned, notwithstanding the fact that the sequences may have deletions or additions in certain positions requiring introduction of gaps to allow alignment of the highest percentage of amino acids or bases. Preferably the sequence are aligned by using 10 or less gaps, ie. the total number of gaps introduced into the two sequences when added together is 10 or less. The length of such gaps is not of particular importance as long as the energy producing activity by OGDH is retained but generally will be no more than 10, and preferably no more than 5 amino acids, or 30 and preferably no more than 15 bases.

The expression "conservative substitutions" as used with respect to amino acids relates to the substitution of a given amino acid by an amino acid having physicochemical characteristics in the same class. Thus where an amino acid in the SEQ ID No 1 has a hydrophobic characterising group, a conservative substitution replaces it by another amino acid also having a hydrophobic characterising group; other such classes are those where the characterising group is hydrophilic, cationic, anionic or contains a thiol or thioether. Such substitutions are well known to those of ordinary skill in the art, i.e. see US 5,380,712 which is incorporated herein by reference, and are only contemplated where the resultant protein has energy producing activity in the presence of low NAD⁺.

As used herein, "substantially complementary" means that two nucleic acid sequences have at least about 65%, preferably about 70%, more preferably about 80%, even more preferably 90%, and most preferably about 98%, sequence complementarity to each other. This means that the primers and probes must exhibit sufficient complementarity to their template and target nucleic acid, respectively, to hybridise under stringent conditions. Therefore, the primer sequences as disclosed in this specification need not reflect the exact sequence of the binding region on the template and degenerate primers can be used. A substantially complementary primer sequence is one that has sufficient sequence complementarity to the amplification template to result in primer binding and second-strand synthesis.

The term "hybrid" refers to a double-stranded nucleic acid molecule, or duplex, formed by hydrogen bonding between complementary nucleotides. The terms "hybridise" or "anneal" refer to the process by which single strands of nucleic acid sequences form double-helical segments through hydrogen bonding between complementary nucleotides.

The term "oligonucleotide" refers to a short sequence of nucleotide monomers (usually 6 to 100 nucleotides) joined by phosphorous linkages (e.g., phosphodiester, alkyl and aryl-phosphate (phosphorothioate), or non-phosphorous linkages (e.g., peptide, sulfamate and others). An oligonucleotide may contain modified nucleotides having modified bases (e.g., 5-methyl cytosine) and modified sugar groups (e.g., 2'-O-methyl ribosyl, 2'-O-methoxyethyl ribosyl, 2'-fluoro ribosyl, 2'-amino ribosyl, and the like). Oligonucleotides may be naturally-occurring or synthetic molecules of double- and single-stranded DNA and double- and single-stranded RNA with circular, branched or linear shapes and optionally including domains capable of forming stable secondary structures (e.g., stem-and-loop and loop-stem-loop structures).

The term "primer" as used herein refers to an oligonucleotide which is capable of annealing to the amplification target allowing a DNA polymerase to attach thereby serving as a point of initiation of DNA synthesis when placed under conditions in which synthesis of primer extension product which is complementary to a nucleic acid strand is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxy ribonucleotide. The primer must be sufficiently long to prime the synthesis of extension products in the presence of the agent for polymerization. The exact lengths of the primers will depend on many factors, including temperature and source of primer. A "pair of bi-directional primers" as used herein refers to one forward and one reverse primer as commonly used in the art of DNA amplification such as in PCR amplification.

The term "probe" refers to a single-stranded oligonucleotide sequence that will recognize and form a hydrogen-bonded duplex with a complementary sequence in a target nucleic acid sequence analyte or its cDNA derivative.

The terms "stringency" or "stringent hybridization conditions" refer to hybridization conditions that affect the stability of hybrids, e.g., temperature, salt concentration, pH, formamide concentration and the like. These conditions are empirically optimised to maximize specific binding and minimize non-specific binding of primer or probe to its target nucleic acid sequence. The terms as used include reference to conditions under which a probe or primer will hybridise to its target sequence, to a detectably greater degree than other sequences (e.g. at least 2-fold over background). Stringent conditions are sequence dependent and will be different in different circumstances. Longer sequences hybridise specifically at higher temperatures. Generally, stringent conditions are selected to be about 5°C lower than the thermal melting point (Tm) for the specific sequence at a defined ionic strength and pH. The Tm is the temperature (under defined ionic strength and pH) at which 50% of a complementary target sequence hybridises to a perfectly matched probe or primer. Typically, stringent conditions will be those in which the salt concentration is less than about 1.0 M Na⁺ ion, typically about 0.01 to 1.0 M Na⁺ ion concentration (or other salts) at pH 7.0 to 8.3 and the temperature is at least about 30°C for short probes or primers (e.g. 10 to 50 nucleotides) and at least about 60°C for long probes or primers (e.g. greater than 50 nucleotides). Stringent conditions may also be achieved with the addition of destabilizing agents such as formamide. Exemplary low stringent conditions or "conditions of reduced stringency" include hybridization with a buffer solution of 30% formamide, 1 M NaCl, 1% SDS at 37°C and a wash in 2x SSC at 40°C. Exemplary high stringency conditions include hybridization in 50% formamide, 1 M NaCl, 1% SDS at 37°C, and a wash in 0.1x SSC at 60°C. Hybridization procedures are well known in the art and are described in e.g. Ausubel et al, Current Protocols in Molecular Biology, John Wiley & Sons Inc., 1994.

One enzyme which has received particular attention in plant-related apoptosis is poly(ADP-ribose) polymerase-1 or PARP-1 (EC 2.4.2.30). PARP-1 is a nuclear enzyme found in most eukaryotes, including vertebrates, plants, arthropods, molluscs, slime moulds, dinoflagellates, fungi and other low eukaryotes with the exception of yeast. PARP-1 catalyzes the transfer of the ADP-ribose moiety of NAD⁺, to a specific subset of nuclear substrates in response to DNA damage. PARP-1 is in fact involved at various levels of the cellular response to DNA damage and is an important regulator of the DNA base excision repair (BER) pathway. As such, PARP-1 is essential for the maintenance of genomic integrity and for survival in response to genotoxic insults. Yet, inhibition of PARP-1 was somehow found to prevent the occurrence of PCD. Notwithstanding the essential role of PARP-1 under normal physiological conditions, high PARP-1 activity will significantly reduce the cellular NAD⁺ pool and, during conditions of oxidative stress and energy depletion, PARP-1 activity compartmentalized to the mitochondria contributes to mitochondrial failure and ultimately to cell death due to energy exhaustion. Thus was established that pharmacological or genetic inhibition of PARP-1 during conditions of cellular stress, e.g. energy failure or oxidative stress, is capable of preventing the occurrence of PCD.

The present inventors have now discovered that 2-oxoglutarate dehydrogenase plays an active role in PCD in plants and that protein levels of 2-oxoglutarate dehydrogenase are upregulated after induction of PCD. 2-Oxoglutarate dehydrogenase (OGDH; EC 1.2.4.2) is a component (E1) of the multi-enzyme 2-oxoglutarate dehydrogenase complex, also known as the α-ketoglutarate dehydrogenase complex (KGDHC). This complex catalyses a critical step of the mitochondrial tricarboxylic acid (TCA) or Krebs cycle wherein 2-oxoglutarate (α-ketoglutarate) is converted to succinyl-CoA, carbon dioxide and NADH. The complex further contains as enzymatic components dihydrolipoamide succinyltransferase (E2) and lipoamide dehydrogenase (E3) and uses thiamine pyrophosphate (TPP) as a cofactor.

The 2-oxoglutarate dehydrogenase complex is capable of producing energy at low NAD^{+,} not only in the form of NADH, but also in the form of succinyl-CoA for phosphorylation of GDP and ADP under conditions of restricted NAD⁺ availability. Without wishing to be bound by theory it is believed that 2-oxoglutarate dehydrogenase activity is capable of alleviating the depletion in the cellular NAD⁺ pool brought about by PARP-1 activity, thereby functioning as a rescue mechanism against energy exhaustion and inhibition of PCD.

As a result, the present inventors have now found a method for controlling PCD in plants more in particular for preventing or delaying PCD in plants. The method consists therein that by increasing the activity of 2-oxoglutarate dehydrogenase (OGDH) in plant cells, such as for instance by increasing the expression of the OGDH gene or by expressing an OGDH-homologous protein encoding transgene, the PCD process in said cells may be effectively stalled or even halted. Instead, by inhibiting the activity of OGDH in plant cells, such as for instance by reducing the expression of the OGDH gene, the PCD process in said cells may be induced.

A method for preventing PCD in plants is for instance desired for improving resistance to environmental stresses in susceptible agricultural, horticultural and ornamental crops. Traditional breeding approaches have yielded limited progress in improving hardness of crop plants to environmental stresses, which are often accompanied by PCD. The tolerance of these plants to stress is important in many regions of agriculture. Tolerance to salinity and drought is in particular crucial in dryland and semi-dryland agriculture in eastern Mediterranean regions. But also in agricultural areas that are subject to seawater incursion or where salt water invades the aquifer, salt stress will decrease crop production. In other regions, tolerance to frost is important especially during early spring growth. Environmental stresses such as frost cause large economic losses in susceptible crops. Resistance to stress is known to be multi-allelic and is often negatively correlated with yield factors. Progress by conventional plant-breeding is therefore often slow or non-rewarding.

A method according to the present invention is very suitable to provide plants with the possibility to endure, at least temporary, the effects of environmental stress, such as for instance frost, for example in the case of strawberry. A method to delay the onset of PCD is particularly suitable in such instances where the PCD inducing events are evanescent environmental plant-stress conditions. However, a method of the invention may also be used to provide plants with a more definite resistance to environmental stress. In these embodiments, a method of the invention is aimed at delaying or halting PCD, which is brought about by increasing the plant's OGDH activity. Alternatively a method of the invention may in fact be used to induce or speed-up PCD in plants, which may be brought about by decreasing OGDH activity therein. By modifying the activity and/or expression of OGDH in the cells of a plant or plant-part, the onset and/or progression of programmed cell death in said plant or plant-part may be effectively accelerated.

Since OGDH is essentially a mitochondrial enzyme, the activity and/or expression of OGDH is preferably modified in the mitochondria of the plant cells.

Modifying the activity or expression is used herein as referring to both increasing and decreasing the activity or expression. Methods of modifying the expression of genes are well known in the art. For increasing or improving gene expression see for example US 5,500,365 and references therein; US 2001/0003849 and references therein; US 6,100,451 and references therein; WO 99/63074 and references therein; WO 99/25852 and references therein, which are incorporated herein by reference.

Methods of modifying the activity and/or expression of 2-oxoglutarate dehydrogenase-homologous protein in the cells of said plant or plant-part may comprise the use of transgenic approaches, mutagenic approaches as well as plant breeding approaches. Besides the conventional plant breeding methods, breeding may be combined with marker-assisted selection of those plants that express the desired traits. Such methods are known as marker-assisted selection and breeding and are well established in the field (Deragon and Landry 1992 *PCR Methods Appl.* 1:175-80; Hospital et al. 1992 *Genetics* 132:1199-210; Visscher et al. 1996 *Genetics* 144:1923-32; McCouch et al. 1997 *Plant Mol Biol.* 35:89-99; Kim et al. 1999 *Mol Cells* 9:265; Dekkers and Hospital 2002 *Nat Rev Genet* 3:22-32; Koebner and Summers 2002 *Cell Mol Biol Lett.* 7:695-702; Masojc 2002 *Cell Mol Biol Lett.* 7:499-509; Peleman and Van der Voort 2003 *Trends Plant Sci*. 8:330-4; Kumar 1999 *Biotechnol Adv*. 17:143-82). Knowledge of the chromosomal position of the OGDH gene(s) may for instance be obtained by mapping quantitative trait loci (QTL) using AFLPs, RAPDs, SSRs, ISSRs, STSs, isozymes, microsatellite and morphological marker genotyping, which are all well within the reach of the skilled person. The use of the markers thus facilitates the identification and subsequent selection of plants with a modified activity and/or expression of 2-oxoglutarate dehydrogenase-homologous protein and the use of such plants in further breeding programs. The present invention thus provides a method for modifying the activity and/or expression of 2-oxoglutarate dehydrogenase-homologous protein in the cells of said plant or plant-part, wherein said method involves marker-assisted selection and/or breeding.

The present invention relates to a plant and/or plant part comprising modified activity and/or expression of 2-oxoglutarate dehydrogenase-homologous protein, such as may for instance be obtained by using a method of marker-assisted selection and breeding.

Another method for modifying the activity and/or expression of 2-oxoglutarate dehydrogenase-homologous protein in the cells of said plant or plant-part may comprise the use of mutagenic approaches. Such approaches are well known in the art and may involve a wide variety of mutation methods. Mutation may either occur naturally or may be induced by treating cells with physical or chemical mutagens. Induction of mutation may for instance be obtained by:
- nuclear (X-ray) or UV irradiation,
- chemical mutagenesis by using such agents as hydroxylamine, ethylmethane-sulphonate, N-nitroso-N-ethylurea (NEU), N-nitroso-N-methylurea (NMU); N-methyl-N'-nitrosoguanidine (NG) or acridine dyes such as proflavine
- insertional mutagenesis, involving for instance the insertion of enhancers in or besides a promoter of the OGDH gene
- site directed mutagenesis for instance guided by the three-dimensional structure of OGDH, amino acid residues situated at the active site entrance, may be targeted for site-directed mutagenesis to modify OGDH activity
- site directed mutagenesis of the OGDH promoter region

Methods for or modifying the activity and/or expression of OGDH or a 2-oxoglutarate dehydrogenase-homologous protein in a plant or plant part according to the invention may involve the production of a specific stable or transient change in the genotype of a plant. Such changes may be thus brought about by chemical, radiological, or spontaneous mutation inducing a structural change in a gene in the genome of said plant, thus, without inserting foreign genetic material in said plant or rearrangement of said gene within the plant. Alternatively, transgenic methods may be used, wherein foreign genetic material is introduced into said plant or wherein the genes within the plant are rearranged.

In transgenic approaches, increasing the expression may also consist of using specific promoters (such as homologous promoters and/or inducible promoters), specific terminators, using introns in the open reading frame or specific enhancers. Further, fusion to strongly expressed homologous genes would be a method to increase expression of the gene of interest.

Trangenic methods would also encompass such methods wherein the (*in situ*) expression of the gene is activated, for instance by replacing the natural promoter by a stronger promoter.

Selection of plant cells which have been transformed with a construct which yields overexpression of 2-oxoglutarate dehydrogenase can easily be selected from non-transformed cells if they also contain a marker gene which gives them a phenotype which can discriminate between transformed and untransformed cells. Such selectable markers can be so-called negative selectable markers (such as antibiotic resistance genes) or positive selectable markers (such as genes for using a hitherto not suitable substrate). Examples of selectable markers are well known to the person skilled in the art.

Plant cells, and thus plants, according to the invention advantageously overproduce a 2-oxoglutarate dehydrogenase protein comprising the amino acid sequence presented in SEQ ID No 1 or a functional variant or derivative thereof, said variant being advantageously at least 50% homologous, more advantageously at least 70% homologous and even more advantageously at least 90% homologous to the 2-oxoglutarate dehydrogenase protein sequence represented in SEQ ID No 1. More preferably the cells and plants overproduce such enzymes comprising sequences having at least 50% identity, more preferably at least 70% identity and most preferably at least 90% identity with the amino acid sequence as described in GenBank accession number NP_201376.

Plant cells of the invention are advantageously transgenic and are transformed by incorporation therein of a nucleic acid molecule capable of expressing a 2-oxoglutarate dehydrogenase-homologous protein. Plant cells, plant-parts or plants according to the invention, however, can also be produced via traditional non-biological mutation selection methods wherein, for example, a chemical or UV-light is employed as the mutagenic agent and selection of plants for overproduction of 2-oxoglutarate dehydrogenase enzyme of required activity is carried out using assays eg. as described below. The level of 2-oxoglutarate dehydrogenase expression can be determined by hybridisation (such as Northern or Western blot techniques or quantitative PCR) or via detection of the enzymatic activity, preferably in mitochondria. Enzymatic activity in mitochondria may for instance be determined by preparing a suitable amount (e.g. 25 µg) of total protein as for instance described in Korthout et al.; FEBS Letters 475 (2000) 139-144) and resuspending the protein in a suitable amount (e.g. 2 mL) of a suitable assay buffer (e.g. 50 mM HEPES, 1 mM MgCl₂, 1 mM CaCl₂, 1 mM EGTA, 0.3% Trition-X100). The reaction for measuring the enzymatic activity may then be initiated with the sequential addition of CoA, NAD+ and 2-oxoglutarate in suitable concentrations (e.g. to final concentrations of 60 µM CoA, 1.25 mM NAD+ and 1 mM 2-oxoglutarate, respectively). The generation of NADH may then be measured, e.g by fluorescence spectroscopy using 340 nm wavelengths for excitation and 460 nm wavelengths for emission.

In an aspect of the invention a polynucleotide construct comprising sequences encoding a 2-oxoglutarate dehydrogenase-homologous protein is provided for use in the invention. Particularly this nucleic acid is recombinant, isolated, enriched and/or cell free and encodes for the proteins described above being or having the aforesaid homology to SEQ ID No 1. This provides nucleic acid sequences coding for the 2-oxoglutarate dehydrogenase-homologous protein, particularly for use in the invention and plant cells of the invention for use in producing plants and plant-parts according to the invention.

By use of mutagenesis techniques, e.g. such as side-directed mutagenesis (SDM), the nucleic acids of the invention may be designed to encode the functional variants of the OGDH proteins of the invention. Oligonucleotides and polynucleotides may also be used as probes and primers to identify further naturally occurring or synthetically produced OGDH proteins using e.g. southern or northern blotting.Preferably the nucleic acid is DNA or RNA, particularly cDNA or cRNA and more preferably is characterised in that where it is a DNA it is a polynucleotide comprising nucleotide sequence having at least 80% identity with the nucleotide sequence as described in GenBank accession number NM_125972, or a sequence having degenerate substitution of codon nucleotides in that sequence, and where it is an RNA it has a complementary sequence wherein T is replaced by U. Preferably the identity is 90% or more, more preferably 95% or more and most preferably 100%. Preferably non-identical parts of the sequences comprise degenerate substitutions.

Most preferred DNA or RNA is that which is capable of hybridizing with one or both strands of the nucleic acid of SEQ ID No 2, and polynucleotide and oligonucleotide fragments thereof of 15 or more contiguous bases, preferably 30 or more and more preferably 100 or more, under high stringency conditions, more preferably being capable of such hybridization with two or more of these polynucleotides or oligonucleotides. Most suitable selections of sequences for performing these hybridizations will be selected from coding regions of SEQ ID No 2.

In another aspect of the invention is provided a polynucleotide construct encoding for a 2-oxoglutarate dehydrogenase-homologous protein, in the form of a vector or an isolated nucleic acid sequence, combined in frame with a promoter, activating or otherwise regulating sequence capable of promoting its expression in plants, either constitutively or locally, *e.g.* in vegetative or root tissues. Conveniently this expression is non-temporal such that the plant tissue is capable of delaying PCD, at all times, continuously.

It will be realised by those skilled in the art that tissue specific regulatory regions, such as tissue specific promoters, may be used where a specific tissue requires control of PCD therein. Examples of tissue specific promoters will be known to those skilled in the art, but may be exemplified by those of WO 97/20057 (incorporated herein by reference) teaching root specific promoters and those of Rocarti et al, being embryo and seed specific. For control in vegetative tissues, tissue specific promoters may be used but constitutive promoters such as CaMv35S and alfalfa (MsH3gl) (see WO 97/20058 incorporated herein by reference) will have useful application.

In another aspect of the invention there is provided a process for producing plant cells overproducing a 2-oxoglutarate dehydrogenase-homologous protein, which comprises the transformation of a plant cell with a nucleic acid molecule according to the invention.

In another aspect of the present invention there is provided a transgenic plant or part thereof comprising recombinant nucleic acid, a vector or construct as described above. Preferred plants may comprise the nucleic acid of the invention in a construct in functional association with promoter, activating or otherwise regulating sequences. Preferred promoters may be tissue specific such that the resultant expression of protein, and thus its effects, are localised to a desired tissue. Promoters with a degree of tissue specificity will be known to those skilled in the art of plant molecular biology.

Methods of producing vectors and constructs capable of being used in the present invention will occur to those skilled in the art in the light of conventional molecular biology techniques. DNA, RNA and vectors containing or encoding for these may be introduced into target cells by methods well known in the field of plant cell transformation. Particularly preferred is the method of introducing the DNA or RNA into any plant tissue or cell-type that can be regenerated or propagated into (whole) plants, more particularly into immature embryo's, pollen cells or leaf disks, using techniques known to the skilled person.

It may be preferred to express the DNA or RNA of the invention throughout the plant, but in the event that tissue specific effect is to be exploited then it will be understood by those skilled in the art that tissue specific promoters, enhancers or other activators should be incorporated into the transgenic cells employed in operative relation with the DNA.

Numerous specific examples of methods used to produce transgenic plants and plant cells by the insertion of cDNA in conjunction with suitable regulatory sequences will be known to those skilled in the art. Plant transformation vectors have been described by Denecke *et al* (1992) EMBO J. 11,2345-2355 and their further use to produce transgenic plants producing trehalose described in US Patent Application Serial No. 08/290,301. EP 0 339 009 and US 5,250,515 describe strategies for inserting heterologous genes into plants. Electroporation of pollen to produce both transgenic monocotyledonous and dicotyledonous plants is described in US 5629183, US 7530485 and US 7350356. Mitochondrial transformation may for instance be achieved by using particle bombardment or gene gun methods.

Further details may be found in reference works such as Recombinant Gene Expression Protocols. (1997) Edit Rocky S. Tuan. Humana Press. ISBN 0-89603-3333; 0-89603-480-1 (all these references being incorporated herein by reference). It will be realised that no particular limitation on the type of transgenic plant cell or plant to be provided is envisaged; all classes of plant, monocot or dicot, may be produced in transgenic form incorporating the nucleic acid of the invention such that OGDH activity in the plant is altered.

A preferred promoter is a chemically inducible promoter, such as the tobacco PR-1a promoter, which can be activated by foliar application of a chemical inducer. Various chemical inducers may be employed to induce expression of the OGDH coding sequence in the plants transformed according to the present invention. In the context of the instant disclosure, "chemical inducers " include chemicals known to be inducers for the tobacco PR-1a promoter in plants, or close derivatives thereof, while other chemical inducers may be used in combination with other chemically inducible promoters.

Polynucleotide constructs for expression of a gene in the plant nucleus preferably comprise appropriate 3' sequences, such as 3' regulatory sequences or transcription terminators, to be operably linked downstream of the heterologous nucleotide sequence. Several such terminators are available and known in the art (*e*.*g*. tm1 from CaMV, potpi II from potato, E9 from rbcS). Any available terminator known to function in plants can be used in the context of this invention. Numerous other sequences can be incorporated into polynucleotide constructs for expression of a DNA molecule described in this invention. These include sequences which have been shown to enhance expression such as intron sequences (*e*.*g*. from Adh1 and bronzel), viral leader sequences (*e.g*. from TMV, MCMV and AMV) and signal sequences (*e.g.* KDEL, At-BRP1a).

The polynucleotide construct comprises a recombinant polynucleotide for overexpression of at least a first gene encoding a OGDH and optionally other genes encoding additional OGDH-complex enzymes. The expression of said OGDH gene from said construct will result in OGDH overproduction in said plant.

The polynucleotide construct of the present invention is preferable constructed such that it comprises at least and in operable linkage a first promoter that is functional in plants, a first gene encoding OGDH, and a terminator. Optionally the polynucleotide may comprise a gene sequence encoding a selectable or screenable marker operably linked to regulatory sequences for expression and optionally a mitochondrial target sequence.

The recombinant polynucleotide gene constructs may be inserted into vectors, which may be commercially available, suitable for transforming into plants and suitable for expression of the gene product in the transformed cells. Preferably used are binary vectors which are useful for plant transformation using *Agrobacterium.*

Although some of the embodiments of the invention may not be practicable at present, for example because some plant species are as yet recalcitrant to genetic transformation, the practising of the invention in such plant species is merely a matter of time and not a matter of principle, because the amenability to genetic transformation as such is of no relevance to the underlying concept of the invention.

Transformation of plant species is now routine for an impressive number of plant species, including both the *Dicotyledoneae* as well as the *Monocotyledoneae.* In principle any transformation method may be used to introduce a polynucleotide construct according to the invention into a suitable ancestor cell. Methods may suitably be selected from the calcium/polyethylene glycol method for protoplast transformation, electroporation of protoplasts; microinjection into plant material, (DNA or RNA-coated) particle bombardment of various plant material, gene gun methods, infection with (non-integrative) viruses, *in planta Agrobacterium tumefaciens* mediated gene transfer by infiltration of adult plants or transformation of mature pollen or microspores (EP 0 301 316) and the like. A preferred method according to the invention comprises *Agrobacterium*-mediated DNA transfer. Especially preferred is the use of the so-called binary vector technology as disclosed in EP 0 120 516 and U.S. Patent 4,940,838.

Transformation techniques for dicotyledons are well known in the art and include Agrobacterium-based techniques and techniques which do not require Agrobacterium. Non-Agrobacterium techniques involve the uptake of exogenous genetic material directly by protoplasts or cells. This can be accomplished by PEG or electroporation mediated uptake, particle bombardment-mediated delivery, or microinjection. Examples of these techniques are described by Paszkowski *et al* (1984) EMBO J 3:2717-22, Potrykus *et al* (1985) Mol. Gen. Genet. 199:169-177, Reich *et al* (1986) Biotechnology 4:1001-4, and Klein *et al* (1987) Nature 327:70-3. In each case the transformed cells are regenerated to whole plants using standard techniques known in the art.

*Agrobacterium*-mediated transformation is a preferred technique for transformation of dicotyledons because of its high efficiency of transformation and its broad utility with many different species. The many crop species which are routinely transformable by *Agrobacterium* include tobacco, tomato, sunflower, cotton, oilseed rape, potato, soybean, alfalfa and poplar (EP 0 317 511 (cotton), EP 0 249 432 (tomato), WO 87/07299 (Brassica), U.S. 4,795,855 (poplar)).

*Agrobacterium* transformation typically involves the transfer of the binary vector carrying the foreign DNA of interest to an appropriate *Agrobacterium* strain which may depend of the complement of vir genes carried by the host *Agrobacterium* strain either on a co-resident Ti plasmid or chromosomally. The transfer of the recombinant binary vector to *Agrobacterium* is accomplished by a triparental mating procedure using *E. coli* carrying the recombinant binary vector, a helper *E. coli* strain which carries a plasmid and which is able to mobilize the recombinant binary vector to the target *Agrobacterium* strain. Alternatively, the recombinant binary vector can be transferred to *Agrobacterium* by DNA transformation (Hofgen and Willmitzer (1988) Nucl. Acids Res. 16:9877).

Transformation of the target plant species by recombinant *Agrobacterium* usually involves co-cultivation of the *Agrobacterium* with explants from the plant and follows protocols well known in the art. Transformed tissue is regenerated on selectable medium carrying the antibiotic or herbicide resistance marker present between the binary plasmid T-DNA borders.

Transformation of most monocotyledon species has now also become routine. Preferred techniques include direct gene transfer into protoplasts using PEG or electroporation techniques, and particle bombardment into callus tissue. Transformations can be undertaken with a single DNA species or multiple DNA species (i.e. co-transformation) and both these techniques are suitable for use with this invention. Co-transformation may have the advantage of avoiding complex vector construction and of generating transgenic plants with unlinked loci for the gene of interest and the selectable marker, enabling the removal of the selectable marker in subsequent generations, should this be regarded desirable. However, a disadvantage of the use of co-transformation is the less than 100% frequency with which separate DNA species are integrated into the genome (Schocher *et al* (1986) Biotechnology 4:1093-6).Also subcellular organelles of a plant may be transformed in a method according to the present invention. For instance a suitable transformation construct may comprise N-terminal signal peptide for targeting to the mitochondrion, such as for instance described in Herman et al. (Plant Science 163 (2002), pp. 1137-1145), and references therein. Usually, if a mitochondrion-targeting sequence is used, the nuclear DNA is transformed such that signal sequence-comprising proteins are produced which signal sequence targets the proteins to the mitochondrion.

The heterologous gene encoding the 2-oxoglutarate dehydrogenase-homologous protein comprised in a polynucleotide construct of the invention may have any suitable origin and may obtained from any source, preferably plant derived, more preferably from the same plant species.

A suitable method comprises the transforming the plant with a nucleotide construct which is capable of producing a fusion protein, said fusion protein comprising a heterologous or homologous OGDH fused to a nucleus-encoded protein of which the expression level is naturally high or elevated and which is mitochondrion-targeted, either intrinsically or by the provision of a suitable signal sequence (targeting signal). The provision of a cleavable or flexible spacer (e.g. a series of Glycine residues) between the individual proteins of the fusion protein will allow the proper folding of both.

Although considered somewhat more recalcitrant towards genetic transformation, monocotyledonous plants are amenable to transformation and fertile transgenic plants can be regenerated from transformed cells or embryos, or other plant material. Presently, preferred methods for transformation of monocots are microprojectile bombardment of embryos, explants or suspension cells, and direct DNA uptake or (tissue) electroporation. Transgenic maize plants have been obtained by introducing the *Streptomyces hygroscopicus bar-*gene, which encodes phosphinothricin acetyltransferase (an enzyme which inactivates the herbicide phosphinothricin), into embryogenic cells of a maize suspension culture by microprojectile bombardment. The introduction of genetic material into aleurone protoplasts of other monocot crops such as wheat and barley has been reported. Wheat plants have been regenerated from embryogenic suspension culture by selecting embryogenic callus for the establishment of the embryogenic suspension cultures. The combination with transformation systems for these crops enables the application of the present invention to monocots.

Monocotyledonous plants, including commercially important crops such as rice, wheat and corn are also amenable to DNA transfer by *Agrobacterium* strains (WO 94/00977; EP 0 159 418; EP 0 856 060).

A method for producing a transgenic plant or plant-part according to the invention may comprise the step of selecting transformed plants or plant-parts. Generally after transformation, plant cells or cell groupings are selected for the transfer with the polynucleotide construct according to the invention, following by steps know to the skilled person by which the transformed material is regenerated into a whole plant and evaluating the transformed plant for the overproduction of OGDH.

Selectable markers, which may be included as a part of the introduced recombinant DNA, are used to select transformed cells (those containing recombinant DNA) over untransformed cells. Examples of suitable markers include genes that provide antibiotic or herbicide resistance. Cells containing the recombinant DNA are capable of surviving in the presence of antibiotic or herbicide concentrations that kill untransformed cells. Examples of selectable marker genes include the bar gene which provides resistance to the herbicide Basta; the nptII gene which confers kanamycin resistance; the hpt gene which confers hygromycin resistance; and the cah gene which gives resistance to cyanamid. An entire plant can be generated from a single transformed plant cell through cell culturing techniques known to those skilled in the art.

A process for obtaining a transgenic OGDH overproducing plant according to the invention may in an alternative embodiment comprise introducing a vector according to the invention into an ancestor plant, and then producing said transgenic OGDH overproducing plant from said ancestor plant.

Yet another alternative embodiment for obtaining a transgenic OGDH overproducing plant according to the invention may comprise introducing a polynucleotide construct according to the invention into a suitable vector and using said vector to transform a plant-part to produce a transformed plant-part, and then regenerating said transgenic OGDH overproducing plant from said transformed plant-part.

Following DNA transfer and regeneration, putatively transformed plants may be evaluated, for instance using Southern analysis, for the presence of the recombinant DNA according to the invention, copy number and/or genomic organization. In addition, or alternatively, expression levels of the newly introduced DNA may be undertaken, using Northern and/or Western analysis, techniques well known to persons having ordinary skill in the art.

Evaluating the transformed plant for the overproduction of OGDH may comprise measuring OGDH levels in plants or plant-parts by methods known to the person of ordinary skill, for instance as described herein.

The following Examples serve to further illustrate the invention, and are not intended to define limitations or restrict the scope of the subject invention.

### EXAMPLE

### Preparation of a nucleotide construct comprising OGDH and expression in plants.

A construct comprising the OGDH-coding sequence is cloned and expressed in *Arabidopsis thaliana,* by means of transformation with *Agrobacterium* essentially as described by Herman et al. (Plant Science 163 (2002), 1137-1145. The skilled person is capable of adapting this method to accomplish these ends as cloning of a gene and achieving expression thereof via an expression vector is well known in the art. Examples of appropriate molecular biological techniques and instructions sufficient to direct persons of skill through many construction, cloning, and expression methodologies are found in Sambrook, et al., Molecular Cloning. A Laboratory Manual, 2nd Ed., Cold Spring Harbor Laboratory Vols. 1-3 (1989), Methods in Enzymology, Vol. 152: Guide to Molecular Cloning Techniques, Berger and Kimmel, Eds., San Diego: Academic Press, Inc. (1987), Current Protocols in Molecular Biology, Ausubel, et al., Eds., Greene Publishing and Wiley-Interscience, New York (1995) ; Plant Molecular Biology. A Laboratory Manual, Clark, Ed., Springer-Verlag, Berlin (1997).

The expression of a nucleotide construct in the transformants is compared against that of control transformants which contain an "empty" expression vector, i.e. a vector comprising a nucleotide without the OGDH-coding sequence.

Successfully transformed seedlings are selected on the basis of antibiotic resistance, for which an antibiotic selection marker is provided in the nucleotide construct and selected seedlings are grown to adult plants.

In a first screening the phenotype of the transgenic OGDH plants is analyzed by determining their improved resistance to environmental stresses. Environmental stresses that are tested include salinity, temperature and/or drought stress and the test results, expressed as the extend of the occurrence of PCD in these plants, are compared to the results obtained by using control plants.

The transgenic OGDH plants are further analyzed for the presence of elevated levels of OGDH-mRNA, elevated levels OGDH protein, elevated OGDH bioactivity levels and/or modulated NAD/NADH levels as compared to the control plants.

The increased resistance towards environmental stresses in these plants, as observed when the levels of 2-oxoglutarate dehydrogenase-homologous protein in the cells of the plants are increased by the transformation process, indicates the inhibition of the PCD process in these plants.

## Claims

1. A method for controlling programmed cell death in a plant or plant-part comprising modifying the activity and/or expression of 2-oxoglutarate dehydrogenase-homologous protein in the cells of said plant or plant-part.

2. Method according to claim 1, wherein said activity and/or expression is up-regulated.

3. Method according to claim 1 or 2, wherein said method comprises the occurrence of at least one mutation in the genotype of said plant or plant-part.

4. Method according to any one of the preceding claims, wherein the promoter of the gene encoding said 2-oxoglutarate dehydrogenase-homologous protein is replaced.

5. Method according to claim 3, wherein said occurrence of at least one mutation does not involve the insertion of foreign genetic material in said plant or rearrangement of genetic material within said plant.

6. Method according to any one of the preceding claims, wherein said activity and/or expression is modified in the mitochondria of said cells.

7. Method according to claim 1, wherein said method involves marker-assisted selection and/or breeding.

8. A plant, wherein the occurrence of programmed cell death is controlled by a method according to any one of the preceding claims.

9. A method for preventing programmed cell death in a plant or plant-part comprising providing the cells of said plant or plant-part with a polynucleotide construct, comprising a recombinant polynucleotide for overexpression of a 2-oxoglutarate dehydrogenase-homologous protein encoding gene, and which polynucleotide comprises in operable linkage:
(a) a promoter that is functional in plants,
(b) said 2-oxoglutarate dehydrogenase-homologous protein encoding gene,
(c) a terminator, and, optionally,
(d) a gene encoding a selectable or screenable trait operably linked to regulatory sequences for expression.

10. Method according to claim 9, wherein said 2-oxoglutarate dehydrogenase-homologous protein encoding gene is a heterologous gene.

11. Method according claim 9 or 10, wherein said promoter is an inducible promoter.

12. Method according to any one of claims 9 to 11, wherein said promoter is a tissue specific promoter.

13. A transgenic plant obtainable by a method according to any of the claims 9 to 12.

14. A polynucleotide construct, comprising a recombinant polynucleotide for overexpression of a 2-oxoglutarate dehydrogenase-homologous protein encoding gene, and which polynucleotide comprises in operable linkage:
(a) a promoter that is functional in plants,
(b) said 2-oxoglutarate dehydrogenase-homologous protein encoding gene,
(c) a terminator, and, optionally,
(d) a gene encoding a selectable or screenable trait operably linked to regulatory sequences for expression.

15. Polynucleotide construct according to claim 14, wherein said 2-oxoglutarate dehydrogenase encoding gene is fused to a mitochondrial signal sequence.

16. Polynucleotide construct according claim 14 or 15, wherein said promoter is an inducible promoter.

17. Polynucleotide construct according to any one of claims 14 to 16, wherein said promoter is a tissue specific promoter.

18. Vector comprising a polynucleotide construct according to any one of claims 14 to 17.

19. An *Agrobacterium* strain or any other microbial strain comprising a vector according to claim 18.

20. Transgenic plant comprising a polynucleotide construct according to any one of claims 14 to 17.

21. Method for producing a transgenic plant comprising introducing into the genome of a plant or plant-part a vector according to claim 18.

22. Method according to claim 21, wherein said vector is introduced into an ancestor plant, and wherein said transgenic plant is produced from said ancestor plant.

23. Method according to claim 22, wherein said vector is introduced into a plant-part to produce a transformed plant-part, and wherein said transgenic plant is regenerated from said transformed plant-part.
